# EUROPEAN PATENT APPLICATION

(11) **EP 1 225 238 A1**
(43) Date of publication of application: **24.07.2002**
(21) Application number: 01310881.6
(22) Date of filing: 24.12.2001
(51) Int. Cl.: C22C 18/00, A61K 9/00

(54) **Zinc based alloy bolus for veterinary use**

(30) Priority: 05.01.2001 GB 0100297
(71) Applicant: CASTEX PRODUCTS LIMITED, High Peak, Derbyshire SK22 3HF (GB)
(72) Inventor: Whitehead, Derek James, Poynton, Stockport SK12 1NT (GB)
(74) Representative: Coleiro, Raymond

(57) **Abstract**

A metal alloy comprising, by weight of the total weight of the alloy:
(a) at least 50wt% of zinc;
(b) from 30 to 49wt% of magnesium; and
(c) up to 6wt% of copper,
and a bolus comprising such an alloy, as well as the use of this alloy in the preparation of a medicament for the treatment or prevention of facial eczema, foot rot or hypomagnesaemia in an animal.

## Description

This invention relates to a zinc based alloy used for the construction of sustained release boli administered orally to ruminant animals.

The incidence of facial eczema is a major animal health problem in New Zealand and has also been observed in South Africa and the USA. The disease is caused by the toxin sporidesmin carried by the spores of the saprophytic fungus *Pithomyces chartarum* which proliferates on pasture in warm and humid weather conditions. Perennial ryegrass is frequently associated with facial eczema. The toxin damages the livers of sheep, cattle and goats when ingested by the animal. The animal becomes photosensitive and develops the facial lesions characteristic of the disease.

A well established protection against facial eczema in sheep and young cattle is the regular dosing of the animal with zinc compounds and in particular zinc oxide; see Munday, *et al*., *New Zealand Veterinary Journal,* (1997), 45, 93-98.

To be effective the animal should receive a daily dose of elemental zinc in excess of 20 mg/kg/day over a period of up to five weeks. It is generally impracticable to administer zinc compounds as repetitive drenches and alternative methods such as dressing the pasture with zinc oxide are inefficient and expensive. Sustained release boli with adequate reserves of zinc and a sufficient lifespan are hence a particularly attractive means of controlling facial eczema. This requirement has resulted in the development and commercial exploitation of sustained release boli based on compacted zinc oxide.

However, even such commercially available zinc oxide boli may not provide particularly high zinc serum levels, at least over a period of a few weeks. Furthermore, they require surface protection with a waterproof coating e.g. paraffin wax, to control the erosion rate and are relatively fragile. Care is hence needed during transport and administration to the animal to avoid damage.

GB-A-2186291 describes magnesium rich alloys for the manufacture of boli intended for the control of magnesium deficiency disease (hypomagnesaemia) in ruminants. The alloys so described form part of the magnesium-aluminium-copper-zinc constitutional system. In this system, magnesium is the major constituent and the zinc content merely serves to increase the density of the bolus. By contrast alloys according to the present invention are zinc rich and form part of the zinc-magnesium-copper-system.

Surprisingly, we now find that a particularly useful alloy composition, for use in the production of, or incorporation into, a bolus for administration particularly to sheep or young cattle is a zinc based alloy additionally comprising a large amount of magnesium and a small amount of copper.

Such boli are inherently robust and also contain the valuable trace elements magnesium and copper which enhance their value to the farmer.

Thus, according to a first aspect (1), the present invention provides a metal alloy comprising, by weight of the total weight of the alloy,
(a) at least 50 wt% of zinc
(b) from 30 to 49 wt% of magnesium; and
(c) up to 6 wt% of copper.

According to a second aspect (2), the invention provides a mixture suitable for pressing into a bolus for administration to an animal, which mixture comprises a metal alloy in accordance with the first aspect (1) and a high density metal such as iron, copper, nickel, zinc, tungsten or alloys of these metals. The mixture may be in a finely divided form, preferably with a particle size from 100 to 300 mesh (50 to 150 µm). Additionally, binders, e.g. graphite, sugar and lactose may be present.

According to a third aspect (3), the invention provides a release device in the form of a bolus or pellet comprising such a mixture.

Other aspects of the invention include:
(4) a method of preparing a pellet comprising casting the abovementioned alloy (1) according to the first aspect of the invention;
(5) a method of preparing a pellet comprising converting the alloy (1) according to the first aspect of the invention to particulate form, filling the particles into a mould or die and compressing the particles to form a coherent body;
(6) an alloy (1) in accordance with the first aspect of the invention for use in the treatment or prevention of facial eczema in an animal; and
(7) use, in the preparation of a medicament for the treatment or prevention of facial eczema in an animal, of an alloy (1) in accordance with the first aspect of the invention.

In the zinc-magnesium-copper system provided by an alloy (1) in accordance with the first aspect of the invention, the zinc is at least the main active ingredient, whilst the magnesium and copper serve to control the rate of corrosion of a pellet or bolus in the rumen juices.

Furthermore, the magnesium content provides a valuable function in aiding the prevention of the deficiency disease "Grass Staggers" (hypomagnesaemia). Similarly the copper content is advantageous in combating copper deficiency diseases which may also be occasioned by large zinc inputs to the animal.

The alloy of the invention may also provide an effective treatment for foot rot, which is a bacterial disease in the feet of sheep and cattle, characterised by ulceration.

In addition, the alloy may comprise additional elements. Prominent among these are the beneficial trace elements selenium and cobalt. Typical additions of these alloys amount to:
Selenium - 0.01 to 0.2 wt%, preferably 0.015 wt%
Cobalt - 0.5 to 1 wt%, preferably 0.75 wt%

On the other hand other elements are preferably absent from such alloys. For example, the alloy is preferably free from aluminium, which has an adverse effect upon corrosion patterns and the physical properties of boli. Thus, the alloy preferably comprises less than 0.5 wt% of aluminium.

In a metal alloy according to aspect (1) of the invention, it is preferred that the total weight of zinc and magnesium is at least 94 wt%, by weight of the total weight of the alloy.

Respective preferred amounts of zinc, magnesium and copper, by weight of the total weight of the alloy, are:
Zinc - 50 to 70 wt%, more preferably 50 to 65 wt%
Magnesium - 30 to 45 wt%
Copper - 0.5 or 1 to 5 or 6 wt%,

In some embodiments the amount of copper is more preferably 2-5 wt%, especially 3 wt%. In other embodiments the amount of copper is 1-3 wt%, and especially 2 wt%.

Particularly effective alloys (A) and (B) consist, by weight of the total weight of the alloy, of:

| | (A) | (B) |
|---|---|---|
| Zinc | 52% | 65% |
| Magnesium | 45% | 32% |
| Copper | 3% | 3% |

These alloys are also effective where the amount of copper in both is only 2 wt%.

Zinc-magnesium-copper alloys to the above specification and containing substantially less than 50 wt% zinc and having densities of less than 3 gm/ml and when used as boli in vivo are prone to increased risk of regurgitation by the animal. Furthermore and particularly when treating lambs of less than 40kg weight there is a limit to the size of bolus which can be accommodated by the animal. There is hence a further difficulty with these lower zinc alloys in achieving a sufficiently high zinc content in the bolus to meet the minimum daily requirement of the animal for avoiding facial eczema. Whereas this may be compensated for by dosing with more than one bolus this is not an economical solution.

Zinc-magnesium-copper alloys to the above specification and containing substantially more than 70 wt% zinc have favourable densities in excess of 3.5 gm/ml. However their dissolution and/or physical characteristics render them unsuitable for use as boli in the cast form. Alloys containing very high zinc e.g. 85 wt% and over are insoluble in the rumen liquor. Alloys of intermediate zinc contents e.g. 70-85 wt% are only sparingly soluble and are excessively brittle and fragment when exposed to corrodant.

The alloys may be fabricated into boli by casting to shape in permanent or sand base or refractory moulds using standard foundry techniques familiar to those skilled in the art. Magnesium is melted in ferrous or refractory crucibles and the requisite amounts of zinc and copper are dissolved in the molten metal. The alloy is inflammable in the molten state and the surface must be protected with inert gas e.g. argon or a fluid cover flux to avoid excessive oxidation. Metal is introduced into the mould cavities by the usual gravity, low pressure or high pressure casting techniques and the castings stripped with care from the moulds after solidification. Alloys embodying the invention may exhibit brittle tendencies due to their high content of the intermetallic compounds which comprise the zinc rich regions of the zinc-magnesium equilibrium system.

The castings may advantageously be heat treated at temperatures of 200-300°C for a period of e.g. 2-16 hours. This stress relieves the castings and modifies their corrosion characteristics.

The corrosion rate of boli embodying the invention may be modified by applying protective coatings to the exterior surfaces of the boli. Thus the selective application of water impermeable materials such as waxes, synthetic and natural resins, e.g. epoxy, polyurethane, polyester, shellac, and baked linseed oil, to the exterior surface of the bolus can result in the corrosion being confined to one or both ends of the cylindrical bolus. By such means a more uniform zinc pay out rate may be achieved throughout the life of the bolus. Similarly the application of close fitting plastic rings to the exterior of the bolus, such as are commonly used to control the erosion of boli employed for the delivery of anthelmintics such as fenbendazole to cattle, may be used to achieve the same object. Such rings are progressively discarded by the animal as the bolus shortens in length. Suitable plastics for the manufacture of rings include polyvinyl chloride, polyurethane, polyethene and other commonly injection moulded or extruded plastic materials.

A zinc alloy bolus suitable for administration to young lambs of up to 30 kg weight may have dimensions about 17 mm diameter x 80 mm long whereas a bolus for young cattle, e.g. 150 kg weight, may have dimensions of approximately 25 mm diameter x 75 mm length.

The suitability of a bolus for administration is governed by its ability to meet the following criteria. In particular, to be effective the animal should receive a daily dose of elemental zinc in excess of 20 mg/kg/day over a period of several weeks. Boli given to sheep should have a relatively high density to avoid regurgitation and be of a size sufficiently small for ease of oral administration. The requirements for a zinc bolus suitable for lambs weighing 30-40 kg may hence typically be listed as follows:
Available zinc content, 40 gm.
Bolus life, 40 days.
Density, 3.0 gm/ml.
Dimensions, 20 mm diameter x 80 mm long.

A further requirement is that at the end of the bolus life no substantial residue should remain in the rumen and in particular no hard masses such as the weights sometimes employed to augment the density of intraruminal boli. The latter may damage the blades of carcass processing machinery. It is also necessary that the bolus should be economical to manufacture particularly in the case of lambs due to the comparatively low value of the animal.

Preferred embodiments of the invention will now be described with reference to the following Examples.

### Examples 1 and 2

Sample boli to compositions A and B above were prepared by melting and alloying magnesium and zinc of 99.9% purity each under flux cover using iron crucibles. Cylindrical boli each 20mm diameter x 80 mm long with rounded ends were gravity cast to shape in iron moulds. The castings were heat treated at 300°C for 8 hours. Boli of composition A had an average weight of 75gm compared to 87gm for composition B.

A proprietary compacted zinc oxide bolus (P) was also included in the tests described below. The bolus was wax coated apart from one end and was of cylindrical shape with dimensions, 20mm diameter x 80mm long. The bolus weighed 80gm and was said to contain 72gm zinc oxide.

The efficacy of zinc alloy boli prepared from respective alloys of the above compositions (A) and (B) as compared with that of the proprietary bolus (P), as a method of introducing zinc to sheep was judged from the following data.

Lambs of an average weight of 39 kg were dosed with a single zinc alloy bolus according to the above composition and put out to graze on autumn pasture. Blood samples were taken at regular intervals, centrifuged and the serum analysed for zinc content using an atomic absorption spectrometer. The following average zinc serum levels were obtained over a 6 week period.

| Serum Zinc Level, (µmol/litre) | | | | | | | |
|---|---|---|---|---|---|---|---|
| Bolus Type | Time (Days) | | | | | | |
| | 0 | 7 | 14 | 21 | 28 | 35 | 42 |
| A | 8.5 | 11.8 | 13.1 | 15.1 | 15.9 | 13.2 | 9.0 |
| B | 10.3 | 13.1 | 15.2 | 15.8 | 12.6 | 10.2 | - |
| P | 8.1 | 9.1 | 9.8 | 10.9 | 11.9 | 10.6 | 8.3 |

It can be seen from these results on the zinc alloy boli that serum zinc levels increased within 7 days of bolus administration and these were maintained over 5 weeks. These levels of serum level zinc pick up from the zinc alloy boli are similar to those that would be experienced with regular dosing with zinc oxide and in these tests indicate that they are superior to those obtained with a commercial zinc oxide bolus. The effectiveness of the zinc alloy bolus in alleviating the incidence of facial eczema may hence be presumed.

## Claims

1. A metal alloy comprising, by weight of the total weight of the alloy:
(a) at least 50 wt% of zinc;
(b) from 30 to 49 wt% of magnesium; and
(c) up to 6 wt% of copper.

2. A metal alloy according to claim 1, wherein the total weight of zinc and magnesium is at least 94 wt%, by weight of the total weight of the alloy.

3. A metal alloy according to either claim 1 or claim 2, comprising:
(a) 50 to 70 wt% of zinc;
(b) 30 to 45 wt% of magnesium; and
(c) 0.5 to 5 wt% of copper.

4. A metal alloy according to any one of claims 1 to 3, additionally comprising a trace element selected from:
(d) 0.01 to 0.02 wt% selenium; and
(e) 0.5 to 1 wt% cobalt,
by weight of the total weight of the alloy.

5. A metal alloy according to any one of claims 1 to 4, which contains less than 0.5 wt% aluminium.

6. A mixture suitable for pressing into a bolus for administration to an animal comprising a metal alloy according to any one of claims 1 to 5, and a high density metal.

7. A release device in the form of a bolus, comprising a mixture according to claim 6.

8. A method of preparing a bolus comprising the step of casting an alloy according to any one of claims 1 to 5.

9. A method of preparing a bolus comprising the steps of:
(i) converting an alloy according to any one of claims 1 to 5 to particulate form;
(ii) filling said particles into a mould or die; and
(iii)comprising the particles to form a coherent body.

10. The use of an alloy according to any one of claims 1 to 5 in a method of treatment of an animal.

11. The use of an alloy according to any one of claims 1 to 5 in the preparation of a medicament for the treatment or prevention of facial eczema in an animal.

12. The use of an alloy according to any one of claims 1 to 5 in the preparation of a medicament for the treatment or prevention of foot rot in an animal.

13. The use of an alloy according to any one of claims 1 to 5 in the preparation of a medicament for the treatment or prevention of hypomagnesaemia in an animal.
